# NEUE EUROPÄISCHE PATENTSCHRIFT

(11) **EP 0 170 775 B2**
(45) Veröffentlichungstag und Bekanntmachung des Hinweises auf die Entscheidung über den Einspruch: **12.10.1994**
(45) Hinweis auf die Patenterteilung: 08.11.1989
(21) Anmeldenummer: 85103730.9
(22) Anmeldetag: 24.12.1982
(51) Int. Cl.: C07D 209/42, C07D 209/52, A61K 31/40

(54) **Derivate bicyclischer Aminosäuren, Verfahren zu ihrer Herstellung, diese enthaltende Mittel und deren Verwendung sowie neue bicyclische Aminosäuren als Zwischenstufen und Verfahren zu deren Herstellung**
Derivatives of bicyclic amino acids, processes for their preparation, agents containing them and their use as well as bicyclic amino acids as intermediates and process for their preparation
Dérivés d'amino-acides bicycliques, procédé pour leur préparation, agents les contenant et leur utilisation ainsi que d'amino-acides bicycliques comme intermédiaires et procédé pour leur préparation

(30) Priorität: 29.12.1981 DE 3151690; 24.03.1982 DE 3210701
(43) Veröffentlichungstag der Anmeldung: 12.02.1986
(62) Teilanmeldung aus: 82112007.8
(73) Patentinhaber: HOECHST AKTIENGESELLSCHAFT, 65926 Frankfurt am Main (DE)
(72) Erfinder: Urbach, Hansjörg, Dr., D-6242 Kronberg/Taunus (DE); Henning, Rainer, Dr., D-6234 Hattersheim am Main (DE); Teetz, Volker, Dr., D-6238 Hofheim am Taunus (DE); Geiger, Rolf, Prof. Dr., D-6000 Frankfurt am Main 50 (DE); Becker, Reinhard, Dr., D-6200 Wiesbaden (DE); Gaul, Holger, D-6251 Niederneisen (DE)

(56) Entgegenhaltungen:
- EP-A- 0 012 401
- EP-A- 0 031 741
- EP-A- 0 037 231
- EP-A- 0 046 953
- EP-A- 0 049 658
- EP-A- 0 050 800
- EP-A- 0 051 301
- Nature, vol. 288, 1980, pp. 280-283
- Br. J. clin. Pharmac. 17, 1984, pp. 233-241

## Beschreibung

Aus der EP-A-0037231 sind cis, endo-konfigurierte Octahydro-indol-2-carbonsäure-Derivate, diese enthaltende Diastereo-merengemische, Verfahren zu ihrer Herstellung und ihre Verwendung als Inhibitoren des Angiotensin-Converting-Enzymes (ACE) bekannt.

Es wurden neue bicyclische Aminosäure-Derivat gefunden, die stark das ACE hemmen und eine lang andauernde, intensive blutdrucksenkende Wirkung besitzen.

Die vorliegende Erfindung betrifft daher neue Derivate der bicyclischen Aminosäuren der Formel I, 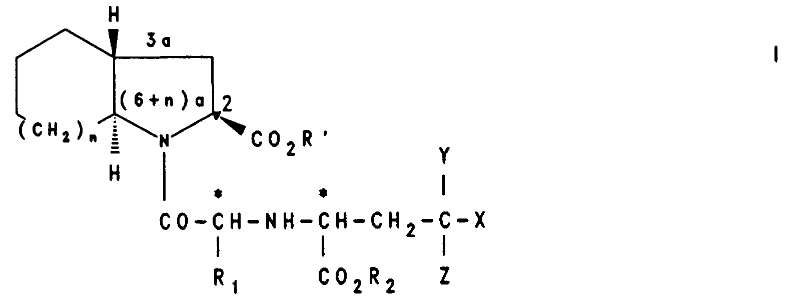 worin
n = 0, 1 oder 2,
R₁ = Wasserstoff, (C1-C3)-Alkyl, (C₂-C₃)-Alkenyl, Benzyl oder 4-Aminobutyl
R₂ = Wasserstoff, (C₁-C₄)-Alkyl, oder Benzyl
R' = Wasserstoff, Benzyl oder tert.-Butyl,
Y = Wasserstoff
Z = Wasserstoff
X = Phenyl

bedeuten, sowie deren physiologisch unbedenkliche Salze,
wobei Verbindungen der Formel (I)
worin
R₁ = Methyl,
R₂ = Wasserstoff, (C₁-C₄)-Alkyl oder Benzyl,
R' = Wasserstoff und
X = Phenyl bedeuten und
n, Y und Z wie oben definiert sind, sowie deren Salze ausgenommen sind.

Als Salze kommen insbesondere in Frage Alkali- und Erdalkalisalze, Salze mit physiologisch verträglichen Aminen und Salze mit anorganischen oder organischen Säuren wie z.B. HCI, HBr, H₂S0₄, Maleinsäure, Fumarsäure.

Alkyl kann geradkettig oder verzweigt sein.

Verbindungen der Formel I besitzen chirale C-Atome in den Positionen C-2, C-3a, C-(6 + n)a, sowie in den mit einem Stern markierten C-Atomen der Seitenkette. Sowohl die R- als auch die S-Konfigurationen an den Zentren sind Gegenstand der Erfindung. Die Verbindungen der Formel I können daher als optische Isomere, als Diastereomere, als Racemate oder als Gemische derselben vorliegen. Bevorzugt sind die Verbindungen der Formel I, in denen die mit einem Stern markierten C-Atome der Seitenkette S-Konfiguration aufweisen.

Bevorzugt sind solche Verbindungen der Formel I, in denen R₁ = Methyl und X = Phenyl bedeuten und solche, in denen R₂ = Wasserstoff oder Ethyl bedeuten.

Gegenstand der Erfindung sind ferner Verfahren zur Herstellung der Verbindungen der Formel I. Eine Verfahrensvariante ist dadurch gekennzeichnet, daß man eine Verbindung der Formel II, 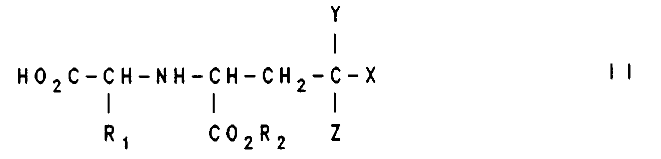 worin Ri, R₂, X, Y und Z die Bedeutung wie in Formel I haben, mit einer Verbindung der Formel III, 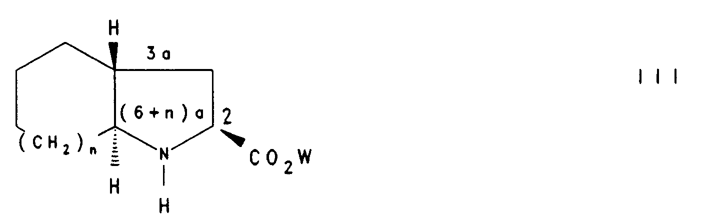 worin
n = 0, 1 oder 2 und
W = einen hydrogenolytisch oder sauer abspaltbaren Rest, insbesondere einen Benzyl- oder einen tert.-Butyl-Rest bedeuten,
nach bekannten Amidbildungsmethoden der Petidchemie umsetzt und anschließend gegebenenfalls durch katalytische Hydrierung oder Säure-Behandlung den Rest W und gegebenenfalls durch zusätzliche Säure-oder Basenbehandlung auch den Rest R₂ abspaltet, wobei jeweils die freien Carbonsäuren erhalten werden.

Weitere Syntheseverfahren zur Herstellung der Verbindungen der Formel I, bestehen darin, daß man eine Verbindung der Formel IV, 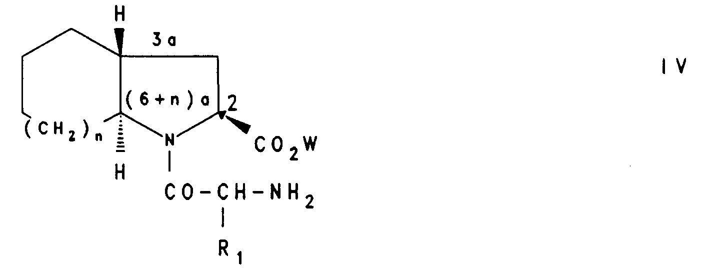 worin n und R₁ die Bedeutungen wie in Formel I besitzen und W die Bedeutung wie in Formel III hat, mit einer Verbindung der Formel V 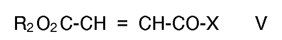 worin R₂ und X die Bedeutungen wie in Formel I besitzen in bekannter Weise in einer Michael-Reaktion (Organikum, 6. Auflage, S. 492, 1967) umsetzt und anschließend gegebenenfalls den Rest W und gegebenenfalls den Rest R₂ wie oben beschrieben abspaltet oder daß man eine Verbindung der obengenannten Formel IV mit einer Verbindung der allgemeinen Formel VI, worin R₂ die Bedeutung wie in Formel I hat, und mit einer Verbindung der allgemeinen Formel VII 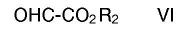 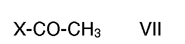 worin X die Bedeutung wie in Formel I hat, in bekannter Weise in einer Mannich-Reaktion (Bull. Soc. Chim. France 1973, S. 625) umsetzt und anschließend gegebenenfalls den Rest W und gegebenenfalls den Rest R₂ wie oben beschrieben unter Bildung der freien Carboxygruppen abspaltet.

Ferner können Verbindungen der Formel auch in der Weise hergestellt werden, daß man eine Verbindung der obengenannten Formel IV gemäß der in J. Amer. Chem. Soc. 93 2897 (1971) beschrieben Verfahrensweise mit einer Verbindung der Formel VIII 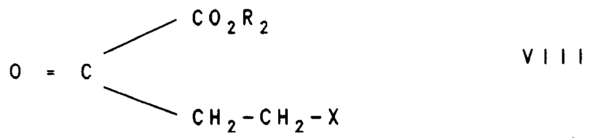 worin R₂ und X die Bedeutungen wie in Formel I besitzen, umsetzt, die erhaltenen Schiff-Basen reduziert und anschließend gegebenenfalls den Rest W und gegebenenfalls den Rest R₂ wie oben beschrieben, unter Bildung der freien Carboxygruppen abspaltet, oder daß man eine gemäß obigen Verfahrensweisen erhaltene Verbindung der Formel I, in der Y und Z zusammen Sauerstoff bedeuten, katalytisch mit Wasserstoff reduziert. Die Reduktion der Schiff-Basen kann katalytisch, elektrolytisch oder mit Reduktionsmitteln wie beispielsweise Natriumborhydrid oder Natriumcyanborhydrid erfolgen.

Die als Ausgangsstoffe zur Herstellung der Verbindungen der Formel 1 verwendeten Verbindungen der Formel II mit Y, Z = Wasserstoff, R₁ = Methyl und R₂ = Methyl oder Äthyl und X = Phenyl sind bekannt (europäische Patentanmeldung Nr. 37 231). Die Verbindungen der Formel II lassen sich nach verschiedenen Verfahrensweisen herstellen. Eine Synthesevariante geht von einem Keton der obengenannten Formel VIII aus, das nach bekannten Verfahrensweisen in einer Mannich-Reaktion mit einer Verbindung der obengenannten Formel VI zusammen mit Aminosäureestern de Formel XXII 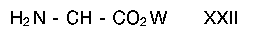 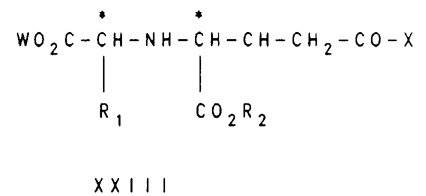 worin R₁ und W die obengenannten Bedeutungen besitzen, zu einer Verbindung der Formel XXIII, worin Ri, R₂, X und W die obengenannten Bedeutungen besitzen, mit der Einschränkung, daß im Falle W = ein hydrogenolytisch abspaltbarer Rest, insbesondere Benzyl bedeutet, R₂ nicht die Bedeutung von W besitzen darf, umsetzt. Spaltet man den Rest W hydrogenolytisch mit Hilfe von beispielsweise Palladium ab, werden Verbindungen der Formel II mit Y, Z = Wasserstoff erhalten. Wird der Rest W mit Säuren wie beispielsweise Trifluoressigsäure oder Salzsäure in einem inerten organischen Lösungsmittel wie beispielsweise Dioxan abgespaltet, erhält man Verbindungen der Formel II mit Y und Z zusammen = Sauerstoff.

Verbindungen der Formel XXIII sind auch durch Michael-Addition einer Verbindung der obengenannten Formel V mit einer Verbindung der obengenannten Formel XXII nach bekannten Verfahrensweisen zugänglich. Bevorzugt eignet sich dieses Verfahren zur Herstellung von solchen Verbindungen der Formel XXIII, in denen R₁ = Methyl, R₂ = Äthyl und X = Aryl bedeuten.

Die Verbindungen der Formel XXIII fallen als Diastereomerengemische an. Bevorzugte Diastereomeren der Formel XXIII sind solche, in denen die mit einem Stern markierten chiralen C-Atome jeweils S-Konfiguration aufweisen. Diese können durch Umkristallisieren oder durch Chromatographie, beispielsweise an Kieselgel, abgetrennt werden. Bei der nachfolgenden Abspaltung des Restes W bleiben die Konfigurationen der chiralen C-Atome erhalten.

Die als Ausgangsstoffe zur Herstellung der Verbindungen der Formel 1 verwendeten Verbindungen der obengenannten Formel IV werden aus den Verbindungen der obengenannten Formel III durch Umsetzen mit einer N-geschützten 2-Aminocarbonsäure der Formel XXIV 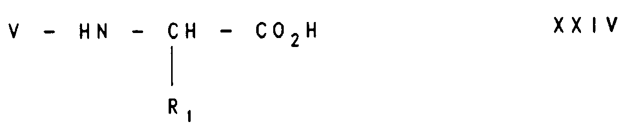 worin V eine Schutzgruppe ist und R₁ die obengenannte Bedeutung besitzt, nach bekannten Verfahrenweisen erhalten. Als Schutzgruppe V, die nach beendeter Reaktion wieder abgespalten wird, kommen beispielsweise die Gruppen Benzyloxycarbonyl oder tert.-Butoxycarbonyl in Frage.

Die Umsetzung einer Verbindung der Formel 11 mit einer Verbindung der Formel 111 zur Herstellung einer Verbindung der Formel 1 erfolgt gemäß einer in der Peptidchemie bekannten Kondensationsreaktion, wobei als Kondensationsmittel beispielsweise Dicyclohexylcarbodiimid und 1-Hydroxybenzotriazol zugesetzt werden. Bei der nachfolgenden hydrogenolytischen Abspaltung des Restes W wird als Katalysator bevorzugt Palladium verwendet, während bei der sauren Abspaltung des Restes W als Säuren bevorzugt Trifluoressigsäure oder Chlorwasserstoff eingesetzt werden.

In den oben beschriebenen Reaktionen zur Herstellung der Verbindungen der Formeln IV und 1 bleiben jeweils die Konfigurationen der Zwischenprodukte an den Brückenkopf C-Atomen 3a und (6 + n)a erhalten.

Fallen die Verbindungen der Formel 1 als Racemate an, können auch diese nach den üblichen Methoden wie beispielsweise über Salzbildung mit optisch aktiven Basen oder Säuren in ihre Enantiomeren gespalten werden.

Die erfindungsgemäßen Verbindungen der Formel 1 liegen als innere Salze vor. Als amphotere Verbindungen können sie Salze mit Säuren oder Basen bilden. Diese Salze werden in üblicher Weise durch Umsetzen mit einem Äquivalent Säure bzw. Base hergestellt.

Die Verbindungen der Formel 1 und deren Salze besitzen lang andauernde, intensive blutdrucksenkende Wirkung. Sie sind starke Hemmer des Angiotensin-Converting-Enzyms (ACE-Hemmer). Sie können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1 - 100 mg, vorzugsweise bei 1 - 40 mg je Einzeldosis bei einem normalgewichtigen erwachsenen Patienten. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken-oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel für die neuen aktiven Verbindungen und entsprechenden physiologisch verträglichen Salze kommen z.B. in Frage: Wasser, physiologische Kochsalzlösungen oder Alkohole, z.B. Äthanol, Propandiol oder Glycerin, daneben auch Zuckerlösungen wie Glukose- oder Mannitlösungen, oder auch eine Mischung aus den verschiedenen genannten Lösungsmitteln.

Die nachfolgenden Beispiele dienen zur Erläuterung der Erfindung, ohne diese auf die stellvertretend genannten Verbindungen zu beschränken:

### Beispiel 1

### N-(1-S-Carbethoxy-3-phenyl-propyl)-S-alanyl-2S, 3aR, 7aS-octahydroindol-2-carbonsäure-benzylester

### a) DL-2ß,3aß,7aa-Octahydroindol-2-carbonsäure-benzylester-Hydrochlorid

Zu einer bei -5 bis 0°C hergestellte Lösung von 1,4 ml Thionylchlorid in 14 ml Benzylalkohol werden bei -10°C bis 0°C 1,4 g DL-2ß,3aß,7aa-Octahydroindol-2-carbonsäure gegeben. Man rührt 1 Stunde bei 0°C und läßt über Nacht bei 20 bis 25 °C stehen. Der Benzylalkohol wird im Hochvakuum bei 50 °C abdestilliert und der Rückstand mit Diisopropyläther digeriert. Man erhält 2,5 g farblose Kristalle vom Fp. 154°C.

b) N-(1-S-Carbethoxy-3-phenyl-propyl)-S-alanyl-2β,3aβ,7aα-octahydroindol-2-carbonsäure-benzylester Zu einer Suspension von 2,16 g N-(1-S-Carbethoxy-3-phenyl-propyl)-S-alanin in 8,6 ml wasserfreiem Dimethylformamid werden 1,06 g 1-Hydroxybenzotriazol, 2,2 g DL-2ß,3aß,7aa-Octahydroindol-2-carbon- säurebenzylester-Hydrochlorid sowie 1,08 ml N-Äthylmorpholin und bei 0 _{°} C 1,7 g Dicyclohexylcarbodiimid gegeben. Nach 3,5 stündigem Rühren bei 20 bis 25 °C wird das Reaktionsgemisch mit 20 ml Essigester verdünnt und der abgeschiedene Dicyclohexylharnstoff abfiltriert. Nach Einengen im Vakuum wird der Rückstand in Ether aufgenommen, zweimal mit gesättigtem wäßrigem Natriumbicarbonat gewaschen, über Natriumsulfat getrocknet und eingedampft. Nach Chromatographie an Kieselgel mit Essigester-Cyclohexan als Laufmittel erhält man 2 blaßgelbe Öle im Verhältnis 1:1, die jeweils ein Isomeres der gewünschten Verbindung enthalten.

'H-NMR-Daten des Isomeren mit 2R, 3aR, 7aS-Konfiguration:
7,35 (s, 5H);
7,2 (s, 5H);
5,18 (s, 2H;
4,55 (d, 1 H);
4,1 (q, 2H);
3,4 - 2,3 (m, 6H);
2,4 - 1,3 (m, 12H);
1,3 (t, 3H);
1,1 (d, 3H).

'H-NMR-Daten des Isomeren mit 2S, 3aS, 7aR- Konfiguration:
7,35 (s, 5H);
7,2 (s, 5H);
5,16 (s, 2H);
4,9 - 4,2 (m, 1 H);
4,2 (q, 2H);
3,9 - 2,4 (m, 6H);
2,4 - 1,4 (m, 12H);
1,25 (d + t, 6H).

c) N-(1-S-Carbethoxy-3-phenyl-propyl)-S-alanyl-2S,3aR,7aS-octahydroindol-2-carbonsäure (Referenzbeispiel)

1,7 g des unter b) erhaltenen Isomeren mit 2S, 3aS, 7aR-Konfiguration werden in 60 ml wasserfreiem Ethanol unter Zusatz von 200 mg Palladium-Kohle (10 %) bei 20 bis 25 °C unter Normaldruck während 2 Stunden hydriert. Der Katalysator wird abfiltriert und das Filtrat eingeengt. Man erhält 1,2 g der Titelverbindung als farblosen Schaum.
¹H-NMR-Daten: 7,2 (s, 5H)
4,4 (m, 1 H);
4,2 (q, 2H);
3,6 - 1,3 (m, 18H);
1,28 (d + t, 6H).

Das Hydrochlorid der obengenannten Verbindung wird als farbloses amorphes Pulver erhalten.

### Beispiel 2 (Referenzbeispiel)

### 2ß,3aa,7aß-octahydroindol-2-carbonsäure

a) 3,4,5,6,7,8-Octahydro-1 H-chinolin-2on

392 g Cyclohexanon und 212 g Acrylnitril werden zusammen mit 20 g Cyclohexylamin und 4 g Eisessig sowie 0,4 g Hydrochinon 4 Stunden bis zu einer Endtemperatur von 200 °C unter Rückfluß erhitzt. Das nach Destillation bei 100 bis 150 ° C / 0,5 mm Hg erhaltene Destillat wird 2 Tage mit 10 ml 50 %iger Essigsäure auf 200 °C erhitzt. Das Reaktionsgemisch wird nach Abkühlen aus Methanol/Wasser umkristallisiert. Zusammen mit dem oben erhaltenen Destillationsrückstand der aus n-Hexan umkristallisiert wird, werden 460 g des Produktes vom Fp. 143 bis 144 ° C erhalten.

b) trans-Octahydro-1 H-chinolin-2on

Ein Gemisch aus 25 g des unter a) erhaltenen Produktes und 70 g Natriumformiat in 120 ml Ameisensäure wird 18 Stunden am Rückfluß zum Sieden erhitzt. Die Reaktionslösung wird mit 20 %igem wäßrigem Natriumhydroxid alkalisch gestellt und mit Essigester extrahiert. Die organische Phase wird mit Natriumsulfat getrocknet und eingedampft. Der Rückstand wird aus Cyclohexan umkristallisiert, wobei das Produkt vom Fp. 152 ° C erhalten wird.

c) 3,3-Dichlor-trans-octahydro-1 H-chinolin-2on

Zu einer Lösung von 19,4 g der unter b) erhaltenen Verbindung und 24,3 g Phosphorpentachlorid in 300 ml wasserfreiem Chloroform wird eine Lösung von 36,4 g Sulfurylchlorid in 40 ml Chloroform bei 20 bis 30 ° C während 30 Minuten zugetropft. Das Gemisch wird 6 Stunden zum Sieden erhitzt und über Nacht bei 20 bis 25 ° C stehen gelassen.

Man neutralisiert mit eiskaltem gesättigtem wäßrigem Kalimcarbonat, extrahiert das Gemisch mit Methylenchlorid, trocknet die organische Phase über Natriumsulfat und engt diese ein. Nach Umkristallisation des Rückstandes aus Ethanol unter Zusatz von Aktivkohle werden 25 g des Produktes von Fp. 195 bis 198 ° C erhalten.

d) 3-Chlor-trans-octahydro-1 H-chinolin-2on

15,6 g der unter c) erhaltenen Verbindung werden in 1 I Ethanol und 9,7 ml Triäthylamin unter Zusatz von Raney-Nickel bei 20 bis 25 ° C unter Normaldruck bis zur Aufnahme von 1 Mol-Äquivalent Wasserstoff hydriert.

Nach Abfiltrieren des Katalysators wird das Filtrat zur Trockene eingeengt und der Rückstand in Essigester aufgenommen. Die organische Phase wird zweimal mit Wasser gewaschen, über Natriumsulfat getrocknet und eingeengt. Der Rückstand wird mit Diisopropyläther verrieben, abgesaugt und getrocknet. Man erhält das Produkt in Form blaßgelber Kristalle von Fp. 115 - 120 ° C.

e) 2ß, 3aa,7aß- Ocatahydroindol-2-carbonsäure

3,75 g der unter d) erhaltenen Verbindung werden zu einer siedenden Lösung von 6,63 g Bariumhydroxid-Octahydrat in 120 ml Wasser gegeben. Nach 4 stündigem Erhitzen unter Rückfluß werden 0,9 ml konz. Schwefelsäure zugesetzt, eine zusätzliche Stunde unter Rückfluß erhitzt, die Reaktionslösung abfiltriert und das Filtrat mittels 1 n Natriumhydroxid auf einen pH-Wert von 6,5 gestellt. Nach Eindampfen der Lösung wird der Rückstand in Ethanol erhitzt, erneut abfiltriert und das Filtrat auf ein kleines Volumen eingeengt. Beim Abkühlen wird ein kristallines 1:1-Gemisch der Titelverbindung mit 2ß,3aß,7aa-Octahydroindol-2-carbonsäure vom Fp. 275 - 276 ° C erhalten.

### eispiel 3 (Referenzbeispiel)

### β,3aβ,6aα-Octahydrocyclopenta[b]pyrrol-2-carbonsäure

a) 1,2,3,4,6,7-Hexahydro-5H-1-pyrind-2-on

Ein Gemisch aus 1 Mol Cyclopentanon, einem Mol Acrylnitril, 0,05 Mol Ammoniumacetat und 3 ml 30 %igem Ammoniak wird in einem Druckgefäß 3 Stunden auf 220 °C erhitzt. Man filtriert das Gemisch über Kieselgel mit Essigester/Cyclohexan (1:1) und kristallisiert den nach Eindampfen des Filtrats erhaltenen Rückstand aus Cyclohexan um. Das Produkt schmilzt bei 118 bis 120 ° C.
¹H-NMR-Daten: 9,4 (s-breit, 1 H);
3,2 - 2,0 (m, 12H).

b) Octahydro-trans-5H-1 pyrind-2-on

Diese Verbindung wird analog der in Beispiel 2 unter b) beschriebenen Verfahrensweise hergestellt. ¹H-NMR-Daten: 7,8 (s-breit, 1 H);
2,9 (s-breit, 1 H);
2,6 - 2,2 (m, 2H);
2,1 - 1,0 (m, 8H).

c) 3,3-Dichlor-octahydro-trans-5H-1-pyrind-2-on

Diese Verbindung wird analog der in Beispiel 2 unter c) beschriebenen Verfahrensweise hergestellt. ¹H-NMR-Daten: 7,9 (s-breit, 1 H);
3,8 (s-breit, 1 H);
3,2 - 2,9 (m, 2H);
2,1 - 1,0 (m, 6H).

d) 3-Chlor-octahydro-trans-5H-1-pyrind-2-on

Diese Verbindung wird analog der in Beispiel 2 unter d) beschriebenen Verfahrensweise hergestellt.
¹H-NMR-Daten: 7,8 (s-breit, 1 H);
4,6 - 4,3 (m, 1 H);
3,3 - 3,0 (m, 2H);
2,1 (d, J = 6 Hz, 2H).

e) 2β,3aβ,6aα-Octahydrocyclopenta[b]pyrrol-2-carbon-säure

Diese Verbindung wird analog der in Beispiel 2 unter e) beschriebenen Verfahrensweise hergestellt.
¹H-NMR-Daten: 4,7 - 4,4 (m, 1 H);
3,0 - 0,9 (m, 10H).

## Patentansprüche (Patentansprüche für folgende(n) Vertragsstaat(en) : BE, CH, DE, FR, GB, IT, LT, LU, NL, SE)

1. Verbindung der Formel I, 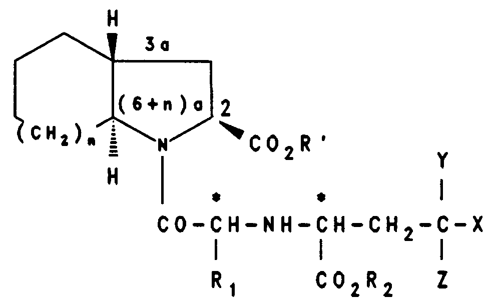 worin
n = 0, 1 oder 2,
R₁ = Wasserstoff, (C₁-C₃)-Alkyl, (C₂-C₃)-Alkenyl, Benzyl oder 4-Aminobutyl
R₂ = Wasserstoff, (C₁-C₄)-Alkyl, oder Benzyl
R' = Wasserstoff, Benzyl oder tert.-Butyl,
Y = Wasserstoff
Z = Wasserstoff
X = Phenyl
bedeuten, sowie deren physliologisch unbedenkliche Salze,
wobei Verbindungen der Formel (I)
worin
R₁ = Methyl,
R₂ = Wasserstoff, (C₁-C₄)-Alkyl oder Benzyl,
R' = Wasserstoff und
X = Phenyl bedeuten und
n, Y und Z wie oben definiert sind, sowie deren Salze ausgenommen sind.

2. Verbindung der Formel 1 gemäß Anspruch 1 dadurch gekennzeichnet, daß die mit einem Stern markierten C-Atome der Seitenkette S-Konfiguration aufweisen.

3. Verbindung der Formel I gemäß einem der Ansprüche 1 oder 2, dadurch gekennzeichnet, daß R₁ = Methyl und X = Phenyl bedeuten.

4. Verbindung der Formel I gemäß einem der Ansprüche 1 bis 3 dadurch gekennzeichnet, daß R₂ = Wasserstoff oder Ethyl bedeutet.

5. Verbindung der Formel I gemäß einem der Ansprüche 1 bis 4, dadurch gekennzeichnet, daß R' Wasserstoff bedeutet.

6. Verfahren zur Herstellung der Verbindung der Formel I gemäß einem der Ansprüche 1 bis 5, dadurch gekennzeichnet,
a) daß man eine Verbindung der Formel II, 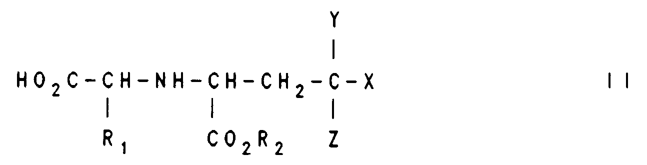 worin Ri, R₂, X, Y und Z die Bedeutungen wie in Formel I haben, mit einer Verbindung der Formel III, 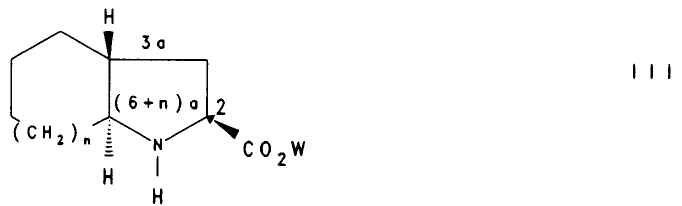 worin
n = 0, 1 oder 2 und
W = einen hydrogenolytisch oder sauer abspaltbaren Rest bedeuten, umsetzt und anschließend gegebenenfalls den Rest W und gegebenenfalls den Rest R₂ unter Bildung der freien Carboxygruppe(n) abspaltet, oder,
b) daß man eine Verbindung der Formel IV, 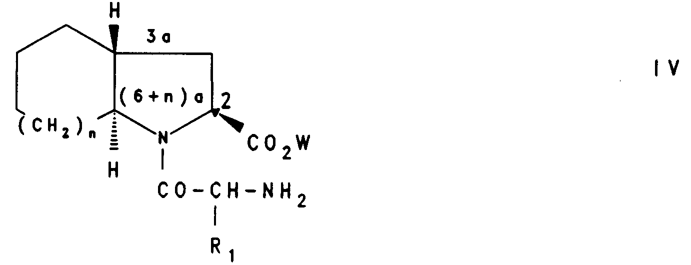 worin n und R₁ die Bedeutungen wie in Formel I und W die Bedeutung wie in Formel III besitzen, mit einer Verbindung der Formel (VIII) 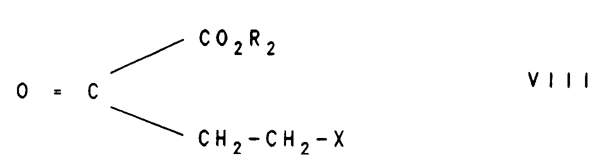 worin R₂ und X die Bedeutungen wie in Formel I besitzen, umsetzt, die erhaltenen Schiff-Basen reduziert und anschließend gegebenenfalls den Rest W und gegebenenfalls den Rest R₂ unter Bildung der freien Carboxygruppe(n) abspaltet, oder
c) eine Verbindung der Formel I, in der Y und Z zusammen Sauerstoff bedeuten, katalytisch mit Wasserstoff reduziert, und die nach (a) - (c) erhaltenen Verbindungen gegebenenfalls in ihre physiologisch verträglichen Salze überführt.

7. Verbindung gemäß einem der Ansprüche 1 bis 5 zur Anwendung als Heilmittel.

8. Verbindung gemäß Anspruch 7 zur Anwendung als Heilmittel bei der Behandlung des Bluthochdrucks.

9. Verbindung gemäß Anspruch 7 oder 8 zur Anwendung in Kombination mit einem Diuretikum.

10. Pharmazeutisches Mittel enthaltend eine Verbindung gemäß einem der Ansprüche 1 bis 5.

11. Mittel gemäß Anspruch 10, dadurch gekennzeichnet, daß es zusätzlich ein Diuretikum enthält.

## Patentansprüche (Patentansprüche für folgende(n) Vertragsstaat(en) : AT)

1. Verfahren zur Herstellung der Verbindungen der Formel I, 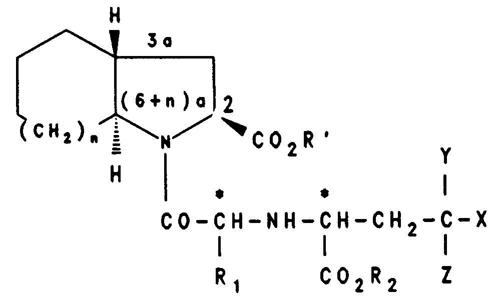 worin
n = 0, 1 oder 2,
R₁ = Wasserstoff, (C₁-C₃)-Alkyl, (C₂-C₃)-Alkenyl, Benzyl oder 4-Aminobutyl
R₂ = Wasserstoff, (C₁-C₄)-Alkyl, oder Benzyl
R' = Wasserstoff, Benzyl oder tert.-Butyl,
Y = Wasserstoff
Z = Wasserstoff
X = Phenyl
bedeuten, und deren physiologisch unbedenklicher Salze,
wobei Verbindungen der Formel (I)
worin
R₁ = Methyl,
R₂ = Wasserstoff, (C₁-C₄)-Alkyl oder Benzyl,
R' = Wasserstoff und
X = Phenyl bedeuten und n, Y und Z wie oben definiert sind, sowie deren Salze ausgenommen sind, dadurch gekennzeichnet,
a) daß man eine Verbindung der Formel II 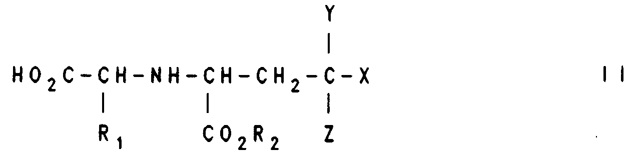 worin Ri, R₂, X, Y und Z die Bedeutungen wie in Formel I haben, mit einer Verbindung der Formel III, 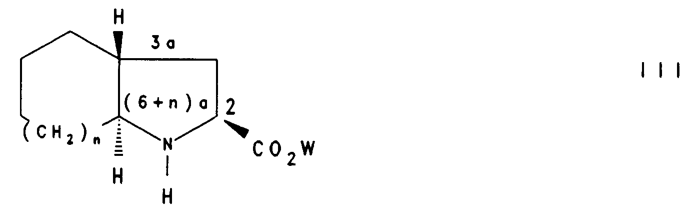 in der H-Atome an den C-Atomen 3a und (6 + n)a zueinander trans-konfiguriert sind, wobei
n = 0, 1 oder 2 und
W = einen hydrogenolytisch oder sauer abspaltbaren Rest bedeuten, umsetzt und anschließend gegebenenfalls den Rest W und gegebenenfalls den Rest R₂ unter Bildung der freien Carboxygruppe(n) abspaltet, oder,
b) daß man eine Verbindung der Formel IV, 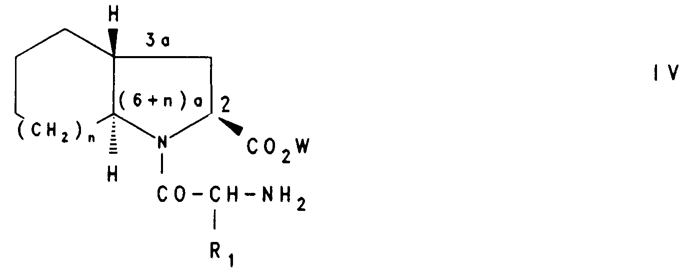 worin n und R₁ die Bedeutungen wie in Formel I und W die Bedeutung wie in Formel III besitzen, mit einer Verbindung der Formel VIII 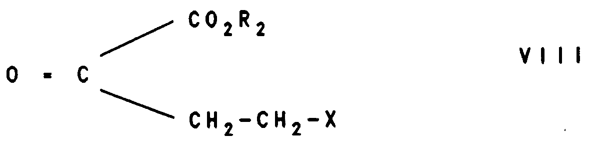 worin R₂ und X die Bedeutungen wie in Formel I besitzen, umsetzt, die erhaltenen Schiff-Basen reduziert und anschließend gegebenenfalls den Rest W und gegebenenfalls den Rest R₂ unter Bildung der freien Carboxygruppe(n) abspaltet, oder
c) eine Verbindung der Formel I, in der Y und Z zusammen Sauerstoff bedeuten, katalytisch mit Wasserstoff reduziert,
und die nach (a) - (c) erhaltenen Verbindungen gegebenenfalls in ihre physiologisch verträglichen Salze überführt.

2. Verfahren zur Herstellung von Verbindungen der Formel 1 gemäß Anspruch 1, dadurch gekennzeichnet, daß die mit einem Stern markierten C-Atome der Seitenkette S-Konfiguration aufweisen.

3. Verfahren zur Herstellung von Verbindungen der Formel I gemäß einem der Ansprüche 1 oder 2, dadurch gekennzeichnet, daß R₁ = Methyl und X = Phenyl bedeuten.

4. Verfahren zur Herstellung von Verbindungen der Formel I gemäß einem der Ansprüche 1 bis 3, dadurch gekennzeichnet, daß R₂ = Wasserstoff oder Ethyl bedeutet.

5. Verfahren zur Herstellung von Verbindungen der Formel I gemäß einem der Ansprüche 1 bis 4, dadurch gekennzeichnet, daß R' Wasserstoff ist.

6. Verfahren zur Herstellung eines pharmazeutischen Mittels enthaltend eine Verbindung der Formel I gemäß einem der Ansprüche 1 bis 5, dadurch gekennzeichnet, daß man diese in eine geeignete Darreichungsform bringt.

7. Verfahren gemäß Anspruch 6, dadurch gekennzeichnet, daß man ein Diuretikum zusetzt.

## Claims (Claims for the following Contracting State(s) : BE, CH, DE, FR, GB, IT, LI, LU, NL, SE)

1. A compound of the formula I 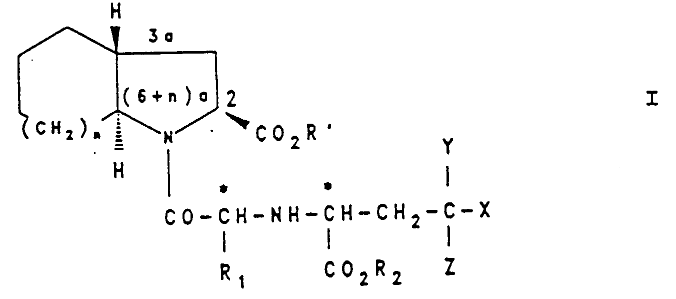 in which
n denotes 0, 1 or 2,
R₁ denotes hydrogen, (C₁-C₃)-alkyl, (C₂-C₃)-alkenyl, benzyl or 4-aminobutyl,
R₂ denotes hydrogen, (C₁-C₄)-alkyl or benzyl,
R' denotes hydrogen, benzyl or tert.-butyl,
Y denotes hydrogen,
Z denotes hydrogen,
X denotes phenyl, or a physiologically acceptable salt thereof, with the exception of a compound of the formula (I) in which
R₁ denotes methyl,
R₂ denotes hydrogen, (C₁-C₄)-alkyl or benzyl,
R' denotes hydrogen and
X denotes phenyl and
n, Y and Z are as defined above, as well as the salts thereof.

2. A compound of the formula I as claimed in claim 1, wherein the carbon atoms labeled with an asterisk in the side chain have the S configuration.

3. A compound of the formula I as claimed in either of claims 1 or 2, wherein R₁ denotes methyl and X denotes phenyl.

4. A compound of the formula I as claimed in any of claims 1 to 3, wherein R₂ denotes hydrogen or ethyl.

5. A compound of the formula I as claimed in any of claims 1 to 4, wherein R' denotes hydrogen.

6. A process for the preparation of a compound of the formula I as claimed in any of claims 1 to 5, which
a) comprises reacting a compound of the formula II 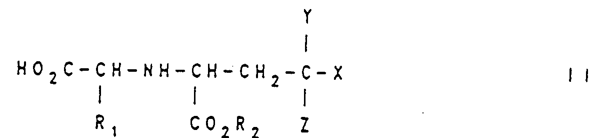 in which R₁ , R₂, X, Y and Z have the meanings as in formula I, with a compound of the formula III 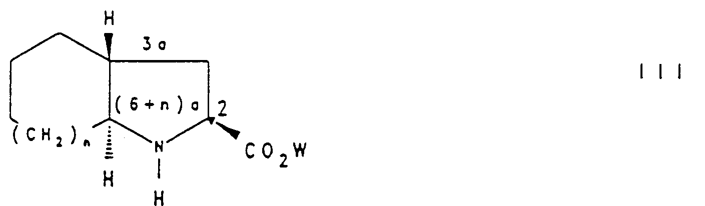 in which
n denotes 0, 1 or 2 and
W denotes a radical which can be cleaved off by hydrogenolysis or by acid, and subsequently cleaving off, if appropriate, the radical W and, if appropriate, the radical R₂ to form the free carboxyl group(s),
or
b) comprises reacting a compound of the formula IV 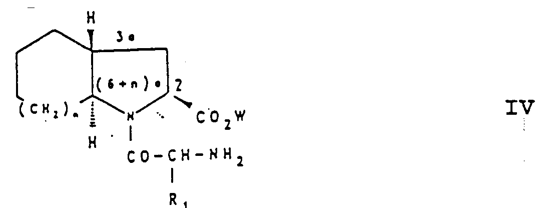 in which n and R₁ have the meanings as in formula I and W has the meaning as in formula III, with a compound of the formula VIII, 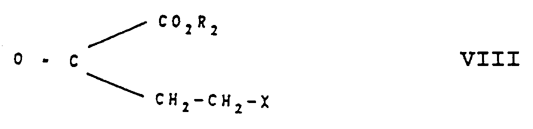 in which R₂ and X have the meanings as in formula I, reducing the Schiff's bases obtained and subsequently cleaving off, if appropriate, the radical W and, if appropriate, the radical R₂ to form the free carboxyl group(s), or
c) catalytically reducing with hydrogen a compound of the formula I in which Y and Z together denote oxygen, and converting the compounds obtained as in (a) - (c), if appropriate, into the physiologically tolerated salts thereof.

7. A compound as claimed in any of claims 1 to 5 for use as a medicine.

8. A compound as claimed in claim 7 for use as a medicine for the treatment of high blood pressure.

9. A compound as claimed in claim 7 or 8 for use in combination with a diuretic.

10. A pharmaceutical agent containing a compound as claimed in any of claims 1 to 5.

11. An agent as claimed in claim 10, which additionally contains a diuretic.

## Claims (Claims for the following Contracting State(s) : AT)

1. A process for the preparation of the compounds of the formula I 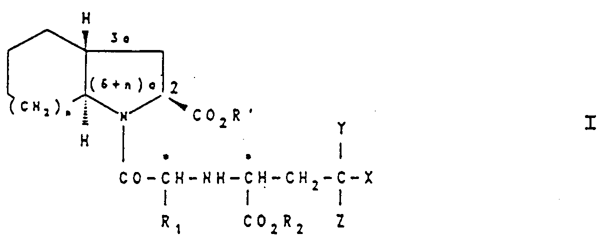 in which
n denotes 0, 1 or 2,
R₁ denotes hydrogen, (C1-C3)-alkyl, (C₂-C₃)-alkenyl, benzyl or 4-aminobutyl,
R₂ denotes hydrogen, (C₁-C₄)-alkyl or benzyl,
R' denotes hydrogen, benzyl or tert.-butyl,
Y denotes hydrogen,
Z denotes hydrogen,
X denotes phenyl, and their physiologically acceptable salts, with the exception of compounds of the formula (I) in which
R₁ denotes methyl,
R₂ denotes hydrogen, (C₁-C₄)-alkyl or benzyl,
R' denotes hydrogen and
X denotes phenyl and
n, Y and Z are as defined above, as well as the salts thereof, which
a) comprises reacting a compound of the formula II 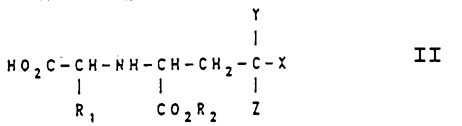 in which R₁ , R₂, X, Y and Z have the meanings as in formula I, with a compound of the formula III 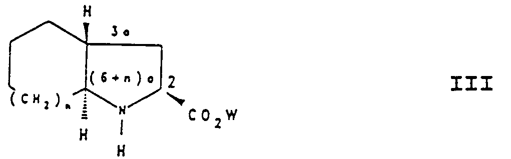 in which the hydrogen atoms on the carbon atoms 3a and (6 + n)a have the trans configuration relative to one another, where
n denotes 0, 1 or 2 and
W denotes a radical which can be cleaved off by hydrogenolysis or by acid, and subsequently cleaving off, if appropriate, the radical W and, if appropriate, the radical R₂ to form the free carboxyl group(s), or
b) comprises reacting a compound of the formula IV 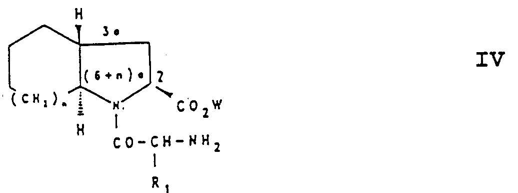 wherein n and R₁ have the meanings as in formula I and W has the meaning as in formula III, with a compound of the formula VIII 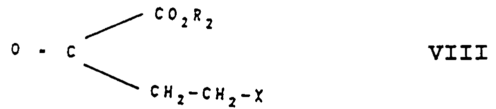 in which R₂ and X have the meanings as in formula I, reducing the Schiff's bases obtained and subsequently cleaving off, if appropriate, the radical W and, if appropriate, the radical R₂ to form the free carboxyl group(s), or
c) catalytically reducing with hydrogen a compound of the formula I in which Y and Z together denote oxygen,
and converting the compounds obtained as in (a) - (c), if appropriate, into the physiologically tolerated salts thereof.

2. A process for the preparation of compounds of the formula I as claimed in claim 1, wherein the carbon atoms labeled with an asterisk in the side chain have the S configuration.

3. A process for the preparation of compounds of the formula I as claimed in any of claims 1 and 2, wherein R₁ denotes methyl and X denotes phenyl.

4. A process for the preparation of the formula I as claimed in any of claims 1 to 3, wherein R₂ denotes hydrogen or ethyl.

5. A process for the preparation of the formula I as claimed in any one of claims 1 to 4, wherein R' denotes hydrogen.

6. A process for preparing a pharmaceutical agent containing a compound of the formula I as in any one of claims 1 to 5, which comprises bringing this compound into a suitable administration form.

7. The process as claimed in claim 6, wherein a diuretic is added.

## Revendications (Revendications pour l'(les) Etat(s) contractant(s) suivant(s) : suivants : BE, CH, DE, FR, GB, IT, LI, LU, NL, SE)

1. Composé de formule 1 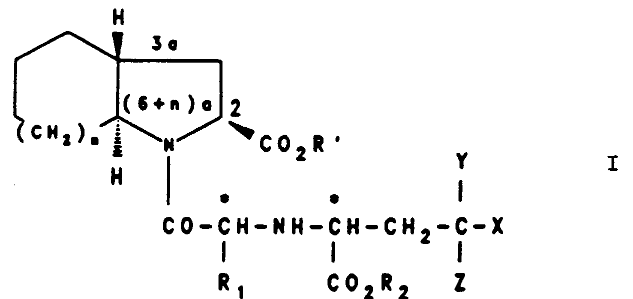 dans laquelle
n est 0, 1 ou 2,
R₁ représente un atome d'hydrogène ou un radical alkyle en C₁-C₃, alcényle en C₂-C₃, benzyle ou 4-aminobutyle;
R₂ représente un atome d'hydrogène ou un groupe alkyle en Ci -C₄ ou benzyle,
R' représente un atome d'hydrogène ou le groupe benzyle ou tert-butyle,
Y représente un atome d'hydrogène,
Z représente un atome d'hydrogène,
X représente le radical phényle,
et sels physiologiquement acceptables de celui-ci, à l'exception des composés de formule I dans lesquels
R₁ représente le groupe méthyle,
R₂ représente un atome d'hydrogène ou un groupe alkyle en Ci -C₄ ou benzyle,
R' représente un atome d'hydrogène et
X représente le radical phényle, et
n, Y et Z sont tels que définis plus haut, ainsi que de leurs sels.

2. Composé de formule I selon la revendication 1, caractérisé en ce que les atomes de carbone de la chaîne latérale qui sont marqués par un astérisque présentent la configuration S.

3. Composé de formule I selon la revendication 1 ou 2, caractérisé en ce que R₁ représente le groupe méthyle et X le groupe phényle.

4. Composé de formule I selon l'une des revendications 1 à 3, caractérisé en ce que R₂ représente un atome d'hydrogène ou le groupe éthyle.

5. Composé de formule I selon l'une des revendications 1 à 4, caractérisé en ce que R' représente un atome d'hydrogène.

6. Procédé pour la préparation du composé de formule I selon l'une des revendications 1 à 5, caractérisé en ce que
a) on fait réagir un composé de formule II 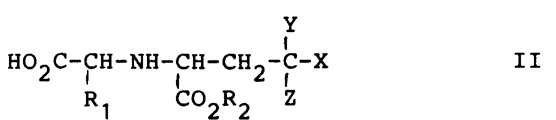 dans laquelle Ri, R₂, X, Y et Z ont les mêmes significations que dans la formule I, avec un composé de formule III 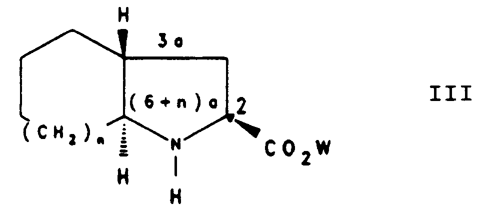 dans laquelle
n est 0, 1 ou 2, et
W représente un reste séparable par voie acide ou par hydrogénolyse,
et ensuite on élimine éventuellement le reste W par hydrogénation ou traitement par un acide, et éventuellement le reste R₂ avec formation du(des) groupe(s) carboxy libre(s) ou
b) on fait réagir un composé de formule IV 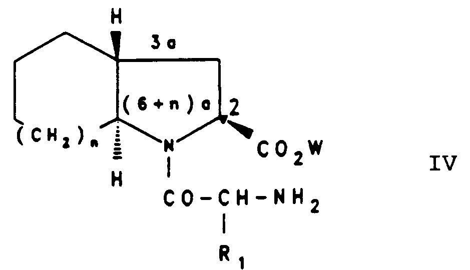
dans laquelle n et R₁ ont les mêmes significations que dans la formule 1 et W a la même signification que dans la formule III,
avec un composé de formule VIII 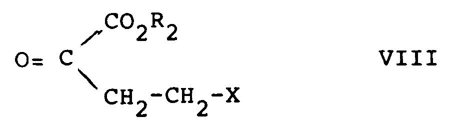 dans laquelle R₂ et X ont les mêmes significations que dans la formule I, on réduit les bases de Schiff obtenues et ensuite, éventuellement on élimine le reste W et éventuellement le reste R₂ avec formation du(des) groupe(s) carboxy libre(s), ou
c) on réduit avec de l'hydrogène, par voie catalytique, un composé de formule I dans lequel Y et Z représentent ensemble un atome d'oxygène, et éventuellement on convertit les composés obtenus selon (a) - (c) en leurs sels physiologiquement acceptables.

7. Composé selon l'une des revendications 1 à 5, pour utilisation en tant que médicament.

8. Composé selon la revendication 7, pour utilisation en tant que médicament dans le traitement de l'hypertension artérielle.

9. Composé selon la revendication 7 ou 8, pour utilisation en association avec un diurétique.

10. Produit pharmaceutique contenant un composé selon l'une des revendications 1 à 5.

11. Produit selon la revendication 10, caractérisé en ce qu'il contient en outre un diurétique.

## Revendications (Revendications pour l'(les) Etat(s) contractant(s) suivant(s) : suivant : AT)

1. Procédé pour la préparation des composés de formule 1 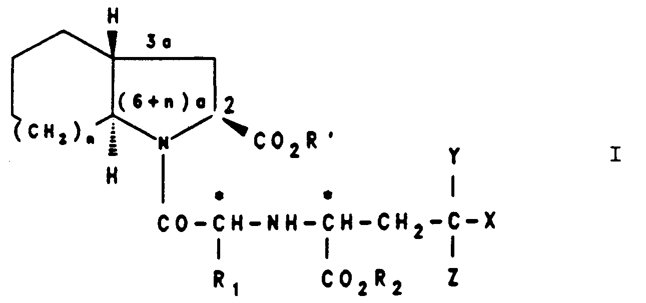 dans laquelle
n est 0, 1 ou 2,
R₁ représente un atome d'hydrogène ou un radical alkyle en C₁-C₃, alcényle en C₂-C₃, benzyle ou 4-aminobutyle;
R₂ représente un atome d'hydrogène ou un groupe alkyle en Ci -C₄ ou benzyle,
R' représente un atome d'hydrogène ou le groupe benzyle ou tert-butyle,
Y représente un atome d'hydrogène,
Z représente un atome d'hydrogène,
X représente le radical phényle, et de leurs sels physiologiquement acceptables, à l'exclusion des composés de formule I dans lesquels
R₁ représente le groupe méthyle,
R₂ représente un atome d'hydrogène ou un groupe alkyle en Ci -C₄ ou benzyle,
R' représente un atome d'hydrogène et
X représente le radical phényle, et
n, Y et Z sont tels que définis plus haut, ainsi que de leurs sels,
caractérisé en ce que
a) on fait réagir un composé de formule II 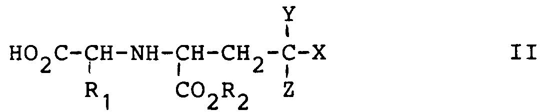 dans laquelle Ri, R₂, X, Y et Z ont les mêmes significations que dans la formule I, avec un composé de formule III 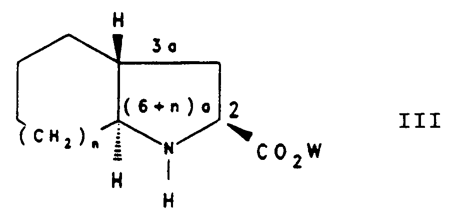 dans laquelle les atomes d'hydrogène sur les atomes de carbone 3a et (6 + n)a sont en configuration trans l'un par rapport à l'autre,
n étant 0, 1 ou 2, et
W représentant un reste séparable par voie acide ou par hydrogénolyse,
et ensuite on élimine éventuellement le reste W par hydrogénation ou traitement par un acide, et éventuellement le reste R₂ avec formation du(des) groupe(s) carboxy libre(s), ou
b) on fait réagir un composé de formule IV 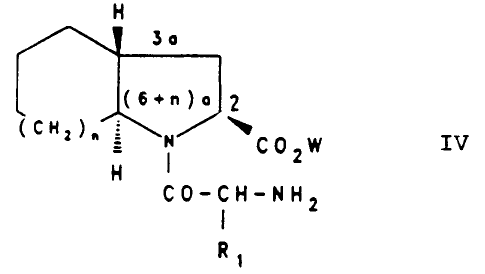 dans laquelle n et R₁ ont les mêmes signififications que dans la formule 1 et W a la même signification que dans la formule III,
avec un composé de formule VIII 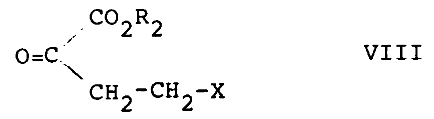 dans laquelle R₂ et X ont les mêmes significations que dans la formule I, on réduit les bases de Schiff obtenues et ensuite, éventuellement on élimine le reste W et éventuellement le reste R₂ avec formation du(des) groupe(s) carboxy libre(s), ou
c) on réduit avec de l'hydrogène, par voie catalytique, un composé de formule I dans lequel Y et Z représentent ensemble un atome d'oxygène,
et éventuellement on convertit les composés obtenus selon (a) - (c) en leurs sels physiologiquement acceptables.

2. Procédé pour la préparation de composés de formule 1 selon la revendication 1, caractérisé en ce que les atomes de carbone de la chaîne latérale qui sont marqués par un astérisque présentent la configuration S.

3. Procédé pour la préparation de composés de formule 1 selon la revendication 1 ou 2, caractérisé en ce que R₁ représente le groupe méthyle et X le groupe phényle.

4. Procédé pour la préparation de composés de formule I selon l'une des revendications 1 à 3, caractérisé en ce que R₂ représente un atome d'hydrogène ou le groupe éthyle.

5. Procédé pour la préparation de composés de formule I selon l'une des revendications 1 à 4, caractérisé en ce que R' est un atome d'hydrogène.

6. Procédé pour la fabrication d'un produit pharmaceutique contenant un composé de formule I selon l'une des revendications 1 à 5, caractérisé en ce qu'on le met sous une forme d'administration appropriée.

7. Procédé selon la revendication 6, caractérisé en ce qu'on ajoute un diurétique.
